# EUROPEAN PATENT APPLICATION

(11) **EP 4 389 069 A1**
(43) Date of publication of application: **26.06.2024**
(21) Application number: 23217296.5
(22) Date of filing: 15.12.2023
(51) Int. Cl.: A61F 2/07, A61F 2/89

(54) **BRANCHABLE STENT GRAFT**

(30) Priority: 21.12.2022 US 202263434207 P; 19.09.2023 US 202318469651
(71) Applicant: Medtronic Vascular Inc., Santa Rosa, CA 95403 (US)
(72) Inventor: DITULLIO, Raymond J., Santa Rosa (US)
(74) Representative: Zimmermann & Partner Patentanwälte mbB

(57) **Abstract**

A branchable stent graft including a tubular body and stents attached to and supporting the tubular body. The tubular body extends along a longitudinal axis and includes proximal and distal ends. The stents include first and second stents. The stents circumferentially extend around the tubular body. The first and second stents are spaced apart along the longitudinal axis of the tubular body. The first stent includes alternating crests and troughs forming peaks and valleys therebetween. The tubular body includes access regions arranged circumferentially spaced around the tubular body. The access regions are confined within the peaks and/or valleys of the first stent. The access regions include peripheries defining openings covered with graft material regions.

## Description

### CROSS-REFERENCE TO RELATED APPLICATIONS

This application claims the benefit of United States Provisional Application 63/434,207 filed December 21, 2022. The disclosure of which are herein incorporated by reference in their entirety.

### TECHNICAL FIELD

The present disclosure relates to a branchable stent graft for use in endovascular procedures to bridge aneurysms and other diseased blood vessels.

### BACKGROUND

Endovascular procedures are minimally invasive techniques to deliver clinical treatments in a patient's vasculature. One example of a clinical treatment used in an endovascular procedure is deployment of a stent graft. A stent graft is an implantable device made of a tube-shaped surgical graft material and an expanding (e.g., self-expanding) stent frame. The stent graft is placed inside a patient's vasculature (e.g., blood vessel) to bridge a diseased blood vessel segment (e.g., an aneurismal, dissected, or torn blood vessel segment), and thereby excluding hemodynamic pressures of blood flow from the diseased blood vessel segment.

The diseased blood vessel segment may extend into vasculature having blood vessel bifurcations or segments of the aorta from which smaller branch arteries extend. For example, thoracic aortic aneurysms may include aneurysms present in the aortic arch where branch arteries (e.g., the left subclavian, left common carotid, or the brachiocephalic arteries) emanate therefrom.

In some cases, deployment of a main stent graft to treat an aneurysm within the aortic arch may cover one or more of the branch arteries. In these cases, the stent graft may be fenestrated in-situ to restore blood flow between the main blood vessel and the one or more covered branch arteries. After the in-situ fenestration is formed, a peripheral stent graft may be anchored to the fenestration and extend into the branch artery.

Alternatively, a branched stent graft may be deployed within the aortic arch and the one or more branch arteries. The branched stent graft may include a coupling extending therefrom and aligning with a branched artery (e.g., left subclavian). As opposed to forming a fenestration in-situ and anchoring a peripheral stent graft thereto, the coupling is formed in the branched stent graft outside of the patient's vasculature and then delivered to the aneurysm treatment site. A secondary stent graft may be connected to the coupling aligned to the branched artery to deploy the secondary stent graft in the branched artery.

### SUMMARY

In an embodiment, a branchable stent graft is disclosed. The branchable stent graft includes a tubular body and stents attached to and supporting the tubular body. The tubular body extends along a longitudinal axis and includes proximal and distal ends. The stents include first and second stents. The stents circumferentially extend around the tubular body. The first and second stents are spaced apart along the longitudinal axis of the tubular body. The first stent includes alternating crests and troughs forming peaks and valleys therebetween. The tubular body includes access regions arranged circumferentially spaced around the tubular body. The access regions are confined within the peaks and/or valleys of the first stent. The access regions include peripheries defining openings covered with graft material regions.

In another embodiment, a branchable stent graft is disclosed. The branchable stent graft includes a tubular body and an interconnected stent attached to and supporting the tubular body. The tubular body extends along a longitudinal axis and includes proximal and distal ends. The interconnected stent forms separated access regions within the interconnected stent arranged circumferentially spaced around the tubular body. The separated access regions include peripheries defining openings covered with graft material regions.

In yet another embodiment, a method of deploying a branchable stent graft in a main artery into a branch artery is disclosed. The method includes delivering a stent graft in a constrained state. The stent graft includes a tubular body extending along a longitudinal axis and one or more stents attached to and supporting the tubular body. The tubular body includes access regions including peripheries defining openings covered with graft material regions. The access regions include a first access region defining a first periphery defining a first opening covered with a first graft material region. The access regions also include a second access region defining a second periphery defining a second opening covered with a second graft material region. The method further includes deploying the stent graft at a deployment site within the main artery by transitioning the stent graft from the constrained state into an expanded state. The method further includes at least partially removing the first graft material region from the first access region and the deployment site. The first access region is closer than the second access region to the branch artery.

### BRIEF DESCRIPTION OF THE DRAWINGS

Figure 1 is a side view of a branched stent graft in an expanded state outside the vasculature of a patient.
Figure 2 is an isolated perspective view of a branch suitable for use with the branched stent graft of Figure 1 in a collapsed state configured to be even with the branched stent graft in a delivery state and/or an initial deployed state.
Figure 3 is an isolated perspective view of the branch of Figure 2 in an expanded state depicting a tapered side extending away from the base of the branch.
Figure 4 is a side view of a fenestrated stent graft in an expanded state outside the vasculature of a patient.
Figure 5 depicts a partial cut away, schematic, side view of an aortic arch branching into a brachiocephalic artery, a left common carotid artery, and a left subclavian artery where a branched stent graft with access regions (e.g., branches and/or fenestrations) is in an initial deployed state within the aortic arch.
Figure 6 depicts a partial cut away, schematic, side view of an abdominal aorta branching into a celiac artery, a superior mesenteric artery (SMA), and right and left renal arteries where a branched stent graft with access regions (e.g., branches and/or fenestrations) is in an initial deployed state within the abdominal aorta.
Figure 7 is a side view of a branched stent graft having a patterned stent formation and branches within the patterned stent formation where the branched stent graft is in an expanded state outside the vasculature of a patient.
Figure 8 is a side view of a branched stent graft having an interconnected stent forming access regions within individual cells thereof.

### DETAILED DESCRIPTION

Embodiments of the present disclosure are described herein. It is to be understood, however, that the disclosed embodiments are merely examples and other embodiments can take various and alternative forms. The figures are not necessarily to scale; some features could be exaggerated or minimized to show details of particular components. Therefore, specific structural and functional details disclosed herein are not to be interpreted as limiting, but merely as a representative basis for teaching one skilled in the art to variously employ the embodiments. As those of ordinary skill in the art will understand, various features illustrated and described with reference to any one of the figures can be combined with features illustrated in one or more other figures to produce embodiments that are not explicitly illustrated or described. The combinations of features illustrated provide representative embodiments for typical applications. Various combinations and modifications of the features consistent with the teachings of this disclosure, however, could be desired for particular applications or implementations.

Directional terms used herein are made with reference to the views and orientations shown in the exemplary figures. A central axis is shown in the figures and described below. Terms such as "outer" and "inner" are relative to the central axis. For example, an "outer" surface means that the surfaces faces away from the central axis, or is outboard of another "inner" surface. Terms such as "radial," "diameter," "circumference," etc. also are relative to the central axis. The terms "front," "rear," "upper" and "lower" designate directions in the drawings to which reference is made.

Unless otherwise indicated, for the delivery system the terms "distal" and "proximal" are used in the following description with respect to a position or direction relative to a treating clinician. "Distal" and "distally" are positions distant from or in a direction away from the clinician, and "proximal" and "proximally" are positions near or in a direction toward the clinician. For the stent-graft prosthesis, "proximal" is the portion nearer the heart by way of blood flow path while "distal" is the portion of the stent-graft further from the heart by way of blood flow path.

The following detailed description is merely exemplary in nature and is not intended to limit the invention or the application and uses of the invention. Although the description is in the context of treatment of blood vessels such as the aorta, coronary, carotid, and renal arteries, the invention may also be used in any other body passageways (e.g., aortic valves, heart ventricles, and heart walls) where it is deemed useful.

The treatment of aortic aneurysms (e.g., those aneurysms bridging one or more branch or peripheral arteries) is complicated. Dedicated off-the-shelf multibranch stent grafts including a main body stent graft and one or more branching stent grafts for bridging down from a main artery into one or more branch arteries have been proposed. These devices may have low patient applicability due to variability in the anatomy of patients. The geometry to accommodate multiple branches on a dedicated device can be complicated to determine. For instance, there may be a significant amount of wire management to support the main body and branching grafts with stents. Procedures to deploy these devices may also be complex. Branching cannulation and/or stenting can be complicated because the devices are susceptible to rotation or axial misalignment.

Physicians can partially deploy an off-the-shelf stent graft on a sterile field and make fenestrations based on patient specific anatomy. However, these procedures include unloading of the stent graft so that it can be modified with a fenestration. Reloading the stent graft is a challenge due to the low profile and high packing density of the stent graft in the radially compressed, delivery state. These modifications are typically labor and time intensive.

Deployment of a fenestrated off-the-shelf stent graft includes deploying a branch stent graft within the fenestration. The branch stent graft may have an unacceptable amount of mobility within the fenestration, thereby causing movement during respiratory cycles, leaking between the fenestration and the branch stent graft, and/or lack of seal between the fenestration and the branch stent graft.

In light of the foregoing, what is needed is a main body stent graft that can be implemented off-the-shelf, be used at a target deployment site with one or more branch arteries, and/or have acceptable mobility. One or more embodiments include a stent graft with a tubular body and stents collectively forming access regions (e.g., branches and/or fenestrations) arranged circumferentially spaced around the tubular body.

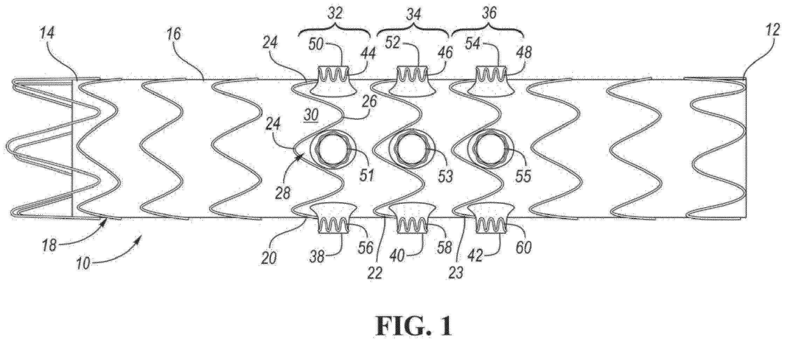

Figure 1 is a side view of branched stent graft 10 in an expanded state outside the vasculature of a patient. Branched stent graft 10 includes distal end 12 and proximal end 14. Branched stent graft 10 includes tubular body 16 and stents 18. Tubular body 16 extends between distal end 12 and proximal end 14. In one or more embodiments, tubular body 16 is formed of a non-permeable graft material (e.g., polyester terephthalate (PET), expanded polyester terephthalate (ePET), polytetrafluoroethylene (PTFE), silicone, or DACRON material). The graft material may be a woven or knit fabric. Stents 18 are attached to and support tubular body 16. Stents 18 circumferentially extend around tubular body 16. Stents 18 include first stent 20, second stent 22, and third stent 23. First stent 20, second stent 22, and third stent 23 are spaced apart from each other along the longitudinal axis of tubular body 16. First stent 20 includes alternating crests 24 and troughs 26 forming valleys 28 and peaks 30 extending therebetween. Crests 24 extend toward proximal end 14 of branched stent graft 10 and troughs 26 extend toward distal end 12 of branched stent graft 10.

Branched stent graft 10 includes first, second, and third circumferential regions 32, 34, and 36. First circumferential region 32 includes branches 38 arranged circumferentially spaced around tubular body 16. Second circumferential region 34 includes branches 40 arranged circumferentially spaced around tubular body 16. Third circumferential region 36 includes branches 42 arranged circumferentially spaced around tubular body 16.

As shown in Figure 1, branches 38 are partially situated within valleys 28 of first stent 20. In this configuration, first stent 20 provides support to branches 38. In other embodiments, branches 38 are confined within valleys 28 of first stent 20 (e.g., beneath a crest and between two troughs) for purposes of alignment with one or more branch arteries and/or conformability of stent graft 10 within the aorta. In an alternative embodiment, branches 38 are confined within peaks 30 instead of valleys 28 (e.g., above a trough and between two crests). This alternative configuration may be used for purposes of alignment and/or conformability.

Branches 38 may be placed at each and every valley 28 or peak 30 of stent graft 10 to increase the likelihood that a branch 38 aligns with a branch artery during deployment. In other embodiments, branches 38 may be placed at every second or third valley 28 or peak 30 of stent graft 10 when radially alignment with a branch artery and a branch 38 may be more easily achieved. In yet other embodiments, branches 38 may be positioned on only one side of stent graft 10 where radially alignment may be maintained between delivery and deployment. In another embodiment, there may be only a single branch 38. Branches 40 and 42 may be similarly placed within regions 34 and 36.

As shown in Figure 1, stents 20, 22, and 23 including branches 38, 40, and 42, respectively, are the fourth, fifth, and sixth stents from proximal end 14 of stent graft 10. Stents 20 and 22 are adjacent and contain branches 38 and 40, respectively. Stents 22 and 23 are adjacent and contain branches 40 and 42, respectively. The configuration shown in Figure 1 may be used with certain anatomy and axial spacing between branch arteries with such anatomy. In other embodiments, the branches may be placed at the first, second, and third stents from proximal end 14 of stent graft 10. In yet other embodiments, alternating stents (e.g., first, third and fifth stents from proximal end 14 of stent graft 10) may include the branches. In one or more embodiments, the tubular body may have only one circumferential region with branches configured to align with one branch artery in a deployed state. In other embodiments, the tubular body may have two circumferential regions with branches configured to align with two branch arteries in a deployed state.

As described in further detail herein, branched stent graft 10 may be deployed at a deployment site (e.g., an aneurysm) within an aortic arch or an abdominal aorta. Depending on the deployment site within the aortic arch, first, second, and/or third circumferential regions 32, 34, and 36 may at least partially align in a longitudinal direction with one or more branch arteries (e.g., the left subclavian, left common carotid, and/or the brachiocephalic arteries) branching from the aortic arch. Depending on the deployment site within the abdominal aorta, first, second, and/or third circumferential regions 32, 34, and 36 may at least partially align in a longitudinal direction with one or more branch arteries (e.g., the celiac artery, the superior mesenteric artery (SMA), and/or the renal arteries) branching from the abdominal artery. One or more of the branches 38, 40, and 42 may be proximate to one or more branch arteries in this aligned position to facilitate locating and extending a branching stent graft into one or more branch arteries. First, second, and/or third circumferential regions 32, 34, and/or 36 may be spaced apart from each other.

Branches 38, 40, and 42 may include tapered sides 44, 46, and 48, respectively. Tapered sides 44, 46, and 48 taper from a wide base at stent graft 10 to narrow openings 50, 52, and 54, respectively. Tapered sides 44, 46, and 48 include stents 56, 58, and 60, respectively, configured to reinforce tapered sides 44, 46, and 48, respectively. Stents 56, 58, and 60 are shown as sinusoidal stents, however, in other embodiments the stents may be formed as an annular ring at the distal end of the branch (e.g., near the free end). In other embodiments, tapered sides 44, 46, and/or 48 do not include stents. Openings 50, 52, and 54 may include a radiopaque (RO) marker (e.g., coil or loop) configured for visualization during an endovascular procedure. The RO marker may also provide structural support to stents 56, 58, and/or 60. Openings 50, 52, and 54 may be covered with graft material regions 51, 53, and 55 such that blood does not perfuse through openings 50, 52, and 54 in an initial deployed state as further described below. In one or more embodiments, the openings of all branches are covered with graft material regions 51, 53, or 55. In one or more embodiments, the graft material regions are formed of a non-permeable graft material. The coverings may be sewn (or otherwise attached) to perimeter of the opening or may be integral with the graft material of the branch. The graft material regions may be spaced apart from tubular body 16 and aligned with the distal ends of the branches.

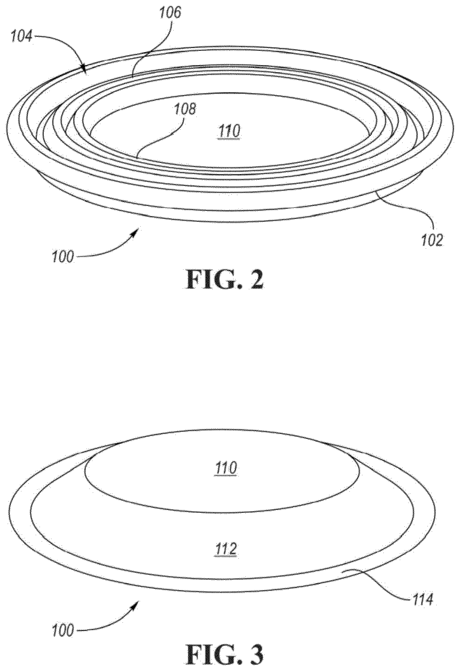

Figure 2 is an isolated perspective view of branch 100 suitable for use with branched stent graft 10 in a collapsed state configured to be even with branched stent graft 10 in a delivery state and an initial deployed state. Branch 100 includes outer periphery 102 configured to be attached (e.g., sewn) to an opening within tubular body 16 of branched stent graft. Collapsed portion 104 includes folds 106 and 108 and is configured to be transitioned from a collapsed state into an expanded state (e.g., tapered side 44, 46, and/or 48) in a final deployed state. Folds 106 and 108 are disposed within periphery 102 and fold 108 is disposed within fold 106. While Figure 2 depicts two folds, a different number of folds (e.g., 1, 3, 4, 5, and 6) may be used in other embodiments. A greater number of folds may accommodate a longer tapered portion for certain applications. Graft material region 110 extends from fold 108 to cover the opening formed within fold 108 such that blood or other liquid does not perfuse through branch 100.

Figure 3 is an isolated perspective view of branch 100 in an expanded state depicting tapered side 112 extending away from base 114 of branch 100. Branch 100 in the collapsed state may follow the curved contour of tubular body 16 to provide a smooth contour to branched stent graft 10 in a delivery state where branched stent graft 10 is in a constrained state and when branched stent graft 10 is in an initial deployed state and the final deployed state. In the initial deployed state, branched stent graft 10 has been delivered to a target deployment site within a patient's vasculature and has been expanded from the constrained state into an expanded state so that the branched stent graft rests against the inner wall of an aortic artery and one or more of the branches at least partially align with one or more branch arteries.

The one or more graft material regions of the one or more at least partially aligned branches may be selectively removed with other graft material regions of branches not at least partially aligned with one or more branch arteries not removed but rather maintained as part of the stent graft to inhibit blood perfusion therethrough. The one or more at least partially aligned branches may be one or more of the most aligned branches. The removal of the one or more graft material regions may be performed using an in-situ fenestration process to obtain access to one or more of the branch arteries, and subsequently stent those graft material regions. In one or more embodiments, the one or more graft material regions are partially removed. In other embodiments, the one or more graft material regions are entirely removed. Partial or entire removal may be premised on the type of branch stent graft extended through the opening and/or the type of branch artery including the branch stent graft. In embodiments where the branches include a stent, the stent may provide a reinforcement to the branch when the graft material region is partially or fully removed. For example, a stent at the distal end of the branch may prevent the in-situ fenestration from being opened larger than desired or may reduce or prevent tearing or fraying of the graft material when a branch stent graft is later inserted and expanded within the opening. In embodiments without stents, stitching or sutures connecting the graft material cover to the branch may serve a similar purpose.

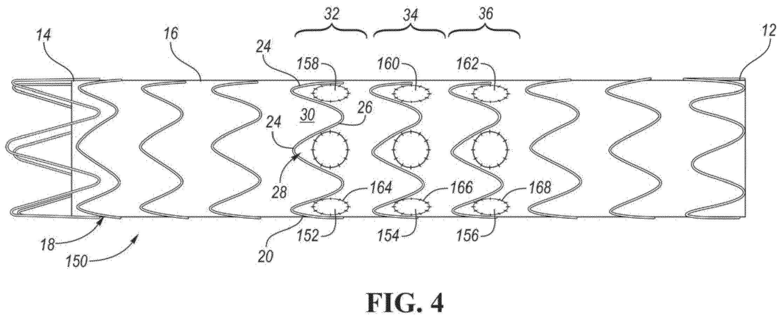

Figure 4 is a side view of fenestrated stent graft 150 in an expanded state outside of the vasculature of a patient. The description above relating to branched stent graft 10 applies to fenestrated stent graft 150 except branches 38, 40, and 42 are replaced with fenestration areas or regions 152, 154, and 156, respectively. In some embodiments, fenestration areas or regions 152, 154, and 156 may be formed during the manufacturing process of fenestrated stent graft 150 (e.g., contrary to forming fenestrations in-situ after delivery within a patient's vasculature and during or after deployment of a stent graft). The openings 164, 166, and 168 formed by fenestration areas or regions 152, 154, and 156 may be covered with graft material regions 158, 160, and 162, respectively, such that blood does not perfuse through the openings 164, 166, and 168 in the initial deployed state and for openings that are not opened thereafter. In other embodiments, the fenestration areas or regions 152, 154, and 156 may be portions of the originally graft material that has been designated for fenestration by creating a closed loop (e.g., a circle) of suture or other filament material (e.g., a metal ring or wire). In these embodiments, there are no openings in the graft material until they are created after deployment and the graft material regions 158, 160, and 162 refer to the original graft material enclosed by the loop. Graft material regions 158, 160, and 162 may be even (e.g., partially or entirely) with the tubular body of fenestrated stent graft 150 in the delivery state and the initial deployed state.

In the initial deployed state, fenestrated stent graft 150 has been delivered to a target deployment site within a patient's vasculature and has been expanded from the constrained state into an expanded state so that fenestrated stent graft 150 rests against the inner wall of an aortic artery and one or more of the covered fenestration areas at least partially align with one or more branch arteries. The one or more graft material regions of the one or more at least partially aligned fenestration areas may be selectively removed with other graft material regions of fenestration areas not at least partially aligned with one or more branch arteries not removed but rather maintained as part of the stent graft to inhibit blood perfusion therethrough. The one or more at least partially aligned fenestration areas may be one or more of the most aligned branches. The removal of the one or more graft material regions may be performed using an in-situ fenestration process to obtain access to one or more of the branch arteries, and subsequently stent those graft material regions. In one or more embodiments, the one or more graft material regions are partially removed. In other embodiments, the one or more graft material regions are entirely removed. Partial or entire removal may be premised on the type of branch stent graft extended through the opening and/or the type of branch artery including the branch stent graft.

The branches and the fenestrations described above may be referred to as non-limiting examples of access regions. A stent graft may include a combination of branches and regions depending on the vasculature of the patient and the types of branching arteries aligning with the branches and the fenestrations.

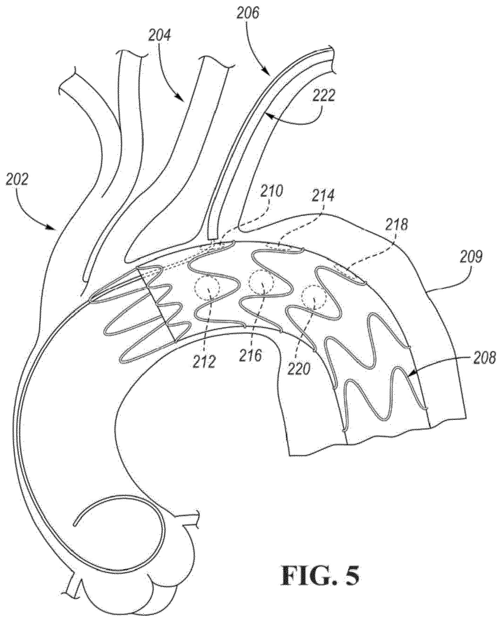

Figure 5 depicts a partial cut away, schematic, side view of aortic arch 200 branching into brachiocephalic artery 202, left common carotid artery 204, and left subclavian artery 206 where stent graft 208 with access regions 210, 212, 214, 216, 218, and 220 (e.g., branches and/or fenestrations) is in an initial deployed state within aortic arch 200. Stent graft 208 spans aneurysm 209. In the initial deployed state, access regions 210, 212, 214, 216, 218, and 220 are covered with stent graft regions configured to resist blood perfusion through the access regions. In the initial deployed state shown in Figure 5, access regions 210 and 212 are positioned within a first circumferential region, access regions 214 and 216 are positioned within a second circumferential region, and access regions 218 and 220 are positioned within a third circumferential region. Access region 210 at least partially aligns with the ostium of left subclavian artery 206. Laser fenestration device 222 (or other fenestration device) is configured to fenestrate (e.g., create an opening in) access region 210 to restore blood perfusion between aortic arch 200 and left subclavian artery 206. The fenestration can then be subsequently stented into left subclavian artery 206 (e.g., using a branch stent graft). In the final deployed state, the other access regions (i.e., access regions 212, 214, 216, 218, and 220) are not opened and remain closed where blood does not perfuse therethrough. Accordingly, in one or more embodiments, access regions can be selectively opened and closed to provide access to branch arteries while maintaining the integrity (e.g., resistance to blood perfusion) of the rest of the stent graft.

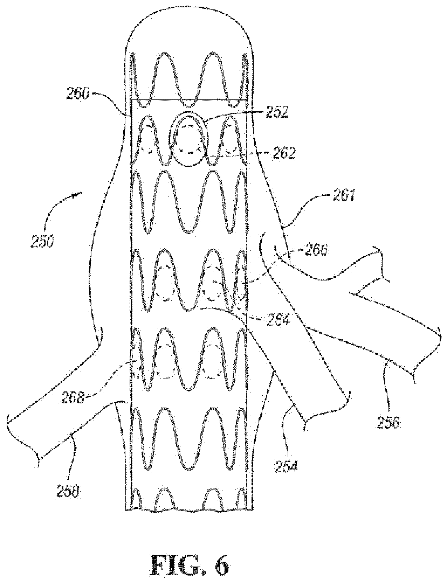

Figure 6 depicts a partial cut away, schematic, side view of abdominal aorta 250 branching into celiac artery 252, superior mesenteric artery (SMA) 254, right and left renal arteries 256 and 258 where stent graft 260 with access regions (e.g., branches and/or fenestrations) is in an initial deployed state within abdominal aorta 250. Stent graft 260 spans aneurysm 261. In the initial deployed state, the access regions are covered with stent graft regions configured to resist blood perfusion through the access regions. In the initial deployed state shown in Figure 6, the access regions are positions in one of three circumferential regions and adjacent to a first, third, or fourth stent graft relative to proximal end 253. Access region 262 at least partially aligns with the ostium of celiac artery 252. Access region 264 at least partially aligns with the ostium of SMA 254. Access regions 266 and 268 at least partially align with the ostia of right and left renal arteries 256 and 258. A fenestration device (e.g., laser fenestration device) is configured to fenestrate access region 262 to restore blood perfusion between abdominal aorta 250 and celiac artery 252; access region 264 to restore blood perfusion between abdominal aorta 250 and SMA 254; and access regions 266 and 268 to restore blood perfusion between abdominal aorta 250 and right and left renal arteries 256 and 258. The fenestrations can then subsequently be stented to deploy branch stent grafts through the fenestrations. In the final deployed state, the access regions other than access regions 262, 264, 266, and 268 are not opened and remain closed where blood does not perfuse therethrough. Accordingly, in one or more embodiments, access regions can be selectively opened and closed to provide access to branch arteries while maintaining the integrity (e.g., resistance to blood perfusion) of the rest of the stent graft.

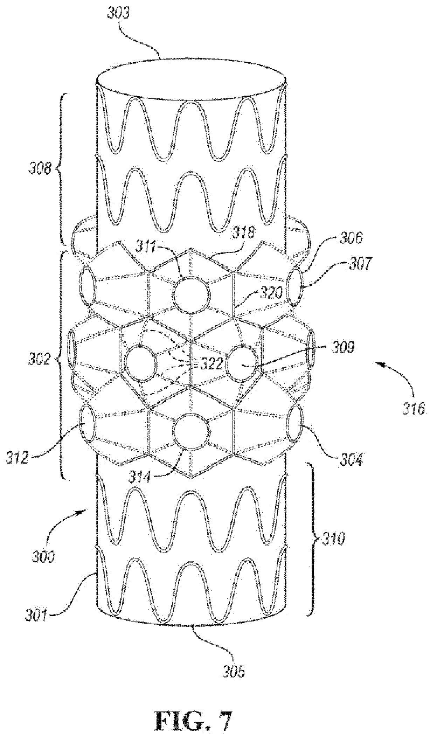

Figure 7 is a side view of branched stent graft 300 having tubular body 301, and interconnected stent 302 and branches 304 within individual cells of interconnected stent 302 where branched stent graft 300 is in an expanded state outside the vasculature of a patient. The individual cells may also include access regions such as openings (e.g., fenestration areas or regions 152, 154, and 156 as shown in Figure 4). The openings may be circular openings having a diameter the size of an inscribed circle within the individual cells or smaller than the inscribed circle. Non-limiting examples of diameters include 4.0 mm, 4.5 mm, 5.0 mm, 5.5 mm, and 6.0 mm. While the individual cells are hexagon shaped as shown in Figure 7, the individual cells may also have different polygonal shapes such as pentagons, heptagons, or octagons. In other embodiments, the openings may be the shape of the cells themselves (e.g., polygonal shaped) so that another shape (e.g., circular shape) does not need to be formed within the cell as another manufacturing step.

As shown in Figure 7, branched stent graft 300 extends between proximal end 303 and distal end 305. Each of branches 304 terminates at an opening at a distal end thereof. Periphery 306 of the opening may include a radiopaque marker (e.g., coil or loop) configured for visualization during an endovascular procedure for implanting branched stent graft 300 within an aorta of a patient. Openings 307 are covered with stent graft regions 309 and 311, for example, configured to resist blood perfusion through the openings. Individual stents may be attached to first and second sides 308 and 310 of branched stent graft 300. While these individual stents are not directly connected to each other (e.g., indirectly connected through the graft material of stent graft 300), interconnected stent 302 is directly interconnected to form an integral stent. As shown in Figure 7, periphery 306 may be reinforced by a stent (e.g., a closed loop stent). In other embodiments, periphery 306 may not include any stent, marker, coil, or loop.

As shown in Figure 7, interconnected stent 302 form access regions separated from each other by portions of interconnected stent 302. As shown in Figure 7, branches 304 (or other access regions as disclosed herein) are located on one of three circumferential regions of branched stent graft 300. While three circumferential regions are shown, there may be fewer or more regions (e.g., 1, 2, 4, 5, or more). At least one circumferential region may align with a branch artery in a deployed state. In some embodiments, at least two circumferential regions may each align with a different branch artery in the deployed state. There may be multiple interconnected stents 302 that are axially spaced along the stent graft 300. As shown in Figure 7, interconnected stent 302 has honeycomb pattern 316 (e.g., hexagonal), although other patterns (e.g., woven pattern, interconnected figure eights, etc.) are also contemplated. The circumferentially extending portions 318 of honeycomb pattern 316 extending circumferentially around tubular body 301 at different longitudinal positions are connected to each other by longitudinally extending struts 320 of honeycomb pattern 316. Longitudinally extending struts 320 are configured to reinforce interconnected stent 302 and to form separated, reinforced access regions.

Optional stent legs 322 are configured to reinforce branches 304. Stent legs 322 extend from periphery 306 (e.g., connect to a stent reinforcing periphery 306) to circumferentially extending portions 318 and longitudinally extending struts 320. Stent legs 322 may be connected to circumferentially extending portions 318 and longitudinally extending struts 320 at corners of the hexagonal pattern to reinforce branches 304. In another embodiment, instead of stent sides 322 for reinforcement, each branch 304 may include an individual stent (e.g., a sinusoidal or zig-zag stent) extending around the branch and between periphery 306 and circumferentially extending portions 318 and longitudinally extending struts 320. In another embodiment, sides of branches 304 do not include any reinforcement (e.g., stent legs 322 or a sinusoidal or zig-zag stent).

While Figure 7 shows branches 304 having peripheral sides extending from proximal ends of the branches, in other embodiments, the separated access regions may be fenestrations bounded by the peripheries where the fenestrations are covered with graft material regions. Separated access regions 304 may include first and second access regions 312 and 314.

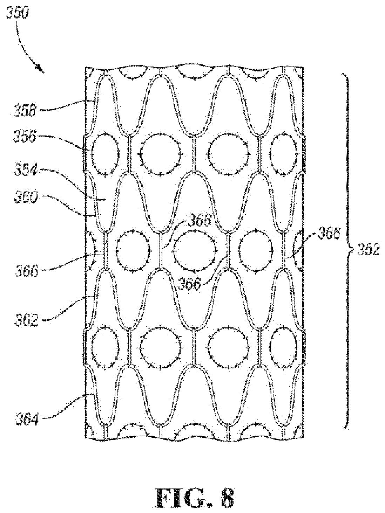

Figure 8 is a side view of branched stent graft 350 having interconnected stent 352 forming access regions 356 within individual cells 354 thereof. Interconnected stent 352 includes circumferentially extending sinusoidal stents 358, 360, 362, and 364. Stents 358 and 360 have mirrored opposing crests and troughs along the longitudinal axis of branched stent graft 350. Struts 366 extend between adjacent crest/trough pairs as shown in Figure 8. Two struts 366 and two sinusoidal stents (e.g., sinusoidal stents 358 and 360) form a boundary of an individual cell 354. Access regions 354 may be fenestrations, branches, individual cell regions, and combinations thereof. As shown in Figure 8, access regions 356 are circular fenestrations that are covered with stent graft material as described herein.

Further disclosed herein is the subject-matter of the following clauses:
Clause 1. A branchable stent graft comprising:
   a tubular body extending along a longitudinal axis and including proximal and distal ends; and
   stents attached to and supporting the tubular body and including first and second stents, the stents circumferentially extending around the tubular body, the first and second stents spaced apart along the longitudinal axis of the tubular body, and the first stent including alternating crests and troughs forming peaks and valleys therebetween,
   the tubular body including access regions arranged circumferentially spaced around the tubular body, the access regions confined within the peaks and/or valleys of the first stent, and the access regions including peripheries defining openings covered with graft material regions.
Clause 2. The branchable stent graft of clause 1, wherein the access regions are branches having peripheral sides extending between proximal and distal ends of the branches, and the distal ends forming the openings.
Clause 3. The branchable stent graft of clause 2, wherein the graft material regions are spaced apart from the tubular body and aligned with the distal ends of the branches.
Clause 4. The branchable stent graft of clause 1, wherein the access regions are branches having peripheral sides nested and collapsed within the openings when the branchable stent graft is in a delivery state and the peripheral sides extend away from the openings when the branchable stent graft is in a deployed state.
Clause 5. The branchable stent graft of clause 1, wherein the access regions are fenestrations bounded by the peripheries and covered with the graft material regions.
Clause 6. The branchable stent graft of clause 5, wherein at least a portion the graft material regions are even with the tubular body.
Clause 7. The branchable stent graft of clause 5, wherein the graft material regions follow the contour of the tubular body.
Clause 8. The branchable stent graft of clause 1, wherein the access regions include first access regions circumferentially spaced around a first circumferential region of the tubular body aligning with a first branch artery of a main artery in a deployed state and second access regions circumferentially spaced around a second circumferential region of the tubular body aligning with a second branch artery of the main artery in the deployed state.
Clause 9. The branchable stent graft of clause 8, wherein the first circumferential region and the second circumferential region are spaced apart.
Clause 10. A branchable stent graft comprising:
   a tubular body extending along a longitudinal axis and including proximal and distal ends; and
   an interconnected stent attached to and supporting the tubular body and forming separated access regions within the interconnected stent arranged circumferentially spaced around the tubular body, and the separated access regions including peripheries defining openings covered with graft material regions.
Clause 11. The branchable stent graft of clause 10, wherein the separated access regions include first and second access regions separated by a portion of the interconnected stent.
Clause 12. The branchable stent graft of clause 10, wherein the interconnected stent has a honeycomb pattern.
Clause 13. The branchable stent graft of clause 10, wherein the separated access regions include branches having peripheral sides extending from proximal ends of the branches, and the distal ends forming openings.
Clause 14. The branchable stent graft of clause 10, wherein the separated access regions are fenestrations bounded by the peripheries and covered with the graft material regions.
Clause 15. The branchable stent graft of clause 10, wherein the separated access regions include first access regions circumferentially spaced around a first circumferential region of the tubular body aligning with a first branch artery of a main artery in a deployed state and second access regions circumferentially spaced around a second circumferential region of the tubular body aligning with a second branch artery of the main artery in the deployed state, and the interconnected stent spanning the first and second circumferential regions.
Clause 16. A method of deploying a branchable stent graft in a main artery branching into a branch artery, the method comprising:
   delivering a branchable stent graft in a constrained state, the stent graft including a tubular body extending along a longitudinal axis and one or more stents attached to and supporting the tubular body, the tubular body including access regions including peripheries defining openings covered with graft material regions, the access regions including a first access region defining a first periphery defining a first opening covered with a first graft material region and a second access region defining a second periphery defining a second opening covered with a second graft material region;
   deploying the branchable stent graft at a deployment site within the main artery by transitioning the branchable stent graft from the constrained state into an expanded state;
   at least partially removing the first graft material region from the first access region and the deployment site, the first access region being closer than the second access region to the branch artery; and
   extending a branch stent graft through the first opening and into the branch artery.
Clause 17. The method of clause 16, wherein the deploying step includes maintaining the first graft material region even with the tubular body in an initial deployed state and moving the first graft material region into a final deployed state where the first graft material region is spaced apart from the tubular body.
Clause 18. The method of clause 17, wherein the first access region has a first peripheral side nested and collapsed within the first opening when the stent graft is in the initial deployed state.
Clause 19. The method of clause 17, further comprising maintaining the second graft material region within the second access region in the final deployed state.
Clause 20. The method of clause 17, wherein the removing step is performed using an in-situ fenestration process.

Further disclosed herein is a branchable stent graft including a tubular body and stents attached to and supporting the tubular body. The tubular body extends along a longitudinal axis and includes proximal and distal ends. The stents include first and second stents. The stents circumferentially extend around the tubular body. The first and second stents are spaced apart along the longitudinal axis of the tubular body. The first stent includes alternating crests and troughs forming peaks and valleys therebetween. The tubular body includes access regions arranged circumferentially spaced around the tubular body. The access regions are confined within the peaks and/or valleys of the first stent. The access regions include peripheries defining openings covered with graft material regions.

While exemplary embodiments are described above, it is not intended that these embodiments describe all possible forms encompassed by the claims. The words used in the specification are words of description rather than limitation, and it is understood that various changes can be made without departing from the spirit and scope of the disclosure. As previously described, the features of various embodiments can be combined to form further embodiments of the invention that may not be explicitly described or illustrated. While various embodiments could have been described as providing advantages or being preferred over other embodiments or prior art implementations with respect to one or more desired characteristics, those of ordinary skill in the art recognize that one or more features or characteristics can be compromised to achieve desired overall system attributes, which depend on the specific application and implementation. These attributes can include, but are not limited to cost, strength, durability, life cycle cost, marketability, appearance, packaging, size, serviceability, weight, manufacturability, ease of assembly, etc. As such, to the extent any embodiments are described as less desirable than other embodiments or prior art implementations with respect to one or more characteristics, these embodiments are not outside the scope of the disclosure and can be desirable for particular applications.

## Claims

1. A branchable stent graft comprising:
a tubular body extending along a longitudinal axis and including proximal and distal ends; and
stents attached to and supporting the tubular body and including first and second stents, the stents circumferentially extending around the tubular body, the first and second stents spaced apart along the longitudinal axis of the tubular body, and the first stent including alternating crests and troughs forming peaks and valleys therebetween,
the tubular body including access regions arranged circumferentially spaced around the tubular body, the access regions confined within the peaks and/or valleys of the first stent, and the access regions including peripheries defining openings covered with graft material regions.

2. The branchable stent graft of claim 1, wherein the access regions are branches having peripheral sides extending between proximal and distal ends of the branches, and the distal ends forming the openings.

3. The branchable stent graft of claim 2, wherein the graft material regions are spaced apart from the tubular body and aligned with the distal ends of the branches.

4. The branchable stent graft of any preceding claim, wherein the access regions are branches having peripheral sides nested and collapsed within the openings when the branchable stent graft is in a delivery state and the peripheral sides extend away from the openings when the branchable stent graft is in a deployed state.

5. The branchable stent graft of any preceding claim, wherein the access regions are fenestrations bounded by the peripheries and covered with the graft material regions.

6. The branchable stent graft of claim 5, wherein at least a portion the graft material regions are even with the tubular body.

7. The branchable stent graft of claim 5 or 6, wherein the graft material regions follow the contour of the tubular body.

8. The branchable stent graft of any preceding claim, wherein the access regions include first access regions circumferentially spaced around a first circumferential region of the tubular body aligning with a first branch artery of a main artery in a deployed state and second access regions circumferentially spaced around a second circumferential region of the tubular body aligning with a second branch artery of the main artery in the deployed state.

9. The branchable stent graft of claim 8, wherein the first circumferential region and the second circumferential region are spaced apart.

10. A branchable stent graft comprising:
a tubular body extending along a longitudinal axis and including proximal and distal ends; and
an interconnected stent attached to and supporting the tubular body and forming separated access regions within the interconnected stent arranged circumferentially spaced around the tubular body, and the separated access regions including peripheries defining openings covered with graft material regions.

11. The branchable stent graft of claim 10, wherein the separated access regions include first and second access regions separated by a portion of the interconnected stent.

12. The branchable stent graft of claim 10 or 11, wherein the interconnected stent has a honeycomb pattern.

13. The branchable stent graft of any one of claims 10 to 12, wherein the separated access regions include branches having peripheral sides extending from proximal ends of the branches, and the distal ends forming openings.

14. The branchable stent graft of any one of claims 10 to 13, wherein the separated access regions are fenestrations bounded by the peripheries and covered with the graft material regions.

15. The branchable stent graft of any one of claims 10 to 14, wherein the separated access regions include first access regions circumferentially spaced around a first circumferential region of the tubular body aligning with a first branch artery of a main artery in a deployed state and second access regions circumferentially spaced around a second circumferential region of the tubular body aligning with a second branch artery of the main artery in the deployed state, and the interconnected stent spanning the first and second circumferential regions.
